# EUROPÄISCHE PATENTSCHRIFT

(11) **EP 4 061 765 B1**
(45) Veröffentlichungstag und Bekanntmachung des Hinweises auf die Patenterteilung: **01.01.2025**
(21) Anmeldenummer: 20806976.5
(22) Anmeldetag: 11.11.2020
(51) Int. Cl.: B67C 7/00, A61L 2/18, A61L 2/22, B67C 3/22

(54) **VORRICHTUNG UND VERFAHREN ZUM BEHANDELN VON BEHÄLTERN**
APPARATUS AND METHOD FOR TREATING CONTAINERS
DISPOSITIF ET PROCÉDÉ DE TRAITEMENT DE CONTENANTS

(30) Priorität: 21.11.2019 DE 102019131494
(43) Veröffentlichungstag der Anmeldung: 28.09.2022
(73) Patentinhaber: KHS GmbH, 44143 Dortmund (DE)
(72) Erfinder: DANELSKI, Alexander, 55543 Bad Kreuznach (DE); LEYENDECKER, Jan, 66839 Schmelz (DE); NIEHR, Thomas, 55583 Bad Münster am Stein Ebernburg (DE)
(86) Internationale Anmeldenummer: PCT/EP2020/081736
(87) Internationale Veröffentlichungsnummer: WO 2021/099192

(56) Entgegenhaltungen:
- WO-A1-01/28863
- WO-A1-2013/004324
- FR-A- 748 104

## Beschreibung

Die Erfindung bezieht sich auf eine Vorrichtung sowie ein Verfahren zum Behandeln von Behältern mit einem Medium, insbesondere zum Sterilisieren von Behältern, insbesondere von Behältern aus Kunststoff, wie zum Beispiel Kunststoffflaschen, und zwar vorzugsweise im Taktbetrieb im Zuge der sterilen Befüllung der Behälter mit einem Getränk, mittels eines taktweise betriebenen Linearfüllers, insbesondere Aseptikfüllers.

Bei der Abfüllung von flüssigen Lebensmitteln, insbesondere Getränken muss aus hygienischer Sicht und aus Gründen der Haltbarkeit des abgefüllten Getränks ein besonderes Augenmerk auf die Sterilisierung gerichtet werden. Voraussetzung für ein steriles Befüllen von Behältern ist dabei die Einhaltung steriler bzw. aseptischer Bedingungen. Ein derartiges steriles Füllen der Behälter erfolgt daher beispielsweise in einer aseptischen Abfüllanlage, nämlich in einem Aseptikfüller.

Dabei werden bekanntlich bereits die noch leeren Behälter in Vorbereitung auf den eigentlichen Füllvorgang einer Sterilisierung unterzogen, wobei im Anschluss daran das eigentliche sterile Befüllen der Behälter erfolgt und das Getränk in die sterilisierten Behälter abgefüllt wird. Die befüllten Behälter wiederum werden direkt anschließend an den Füllvorgang in einem in der Regel von der aseptischen Abfüllanlage ferner umfassten Verschließer unter weiterhin sterilen Bedingungen mit ebenfalls sterilisierten, zugeführten Behälterverschlüssen verschlossen.

Zum Sterilisieren von Flaschen, Dosen oder dergleichen Behältern sind dabei Verfahren unter Verwendung von Wasserstoffperoxid (H₂O₂) enthaltenden Sterilisationsmedien bekannt, d. h. unter Verwendung eines Sterilisationsmediums, welches beispielsweise Wasserstoffperoxid in Mischung mit heißer steriler Luft enthält. Bei diesen Verfahren, die z. B. zum Sterilisieren von Behältern für Getränke, aber auch zum Sterilisieren von Behältern oder Verpackungen für andere Produkte, wie z. B. Arzneimittel, verwendet werden, wird beim Einbringen des heißen H₂O₂-Sterilisationsmediums an der Innenfläche des kühleren Behälters durch Kondensation ein H₂O₂-Kondensationsfilm gebildet, der dann in einer darauffolgenden Aktivierungsphase durch Einbringen eines sterilen heißen gas- und/oder dampfförmigen Aktivierungsmediums, beispielsweise durch Einbringen von heißer steriler Luft in der Weise aktiviert wird, dass durch Zerfall von H₂O₂ freie Sauerstoffradikale entstehen, die mit vorhandenen Keimen und Verunreinigungen reagieren und zur Sterilisation der Behälter führen. Beispielsweise beschreibt die WO 01/28863 A1 ein derartiges Sterilisationsverfahren zur Sterilisation von PET-Flaschen unter Verwendung von H₂O₂.

Bekannt ist auch, dass bei den erwähnten Verfahren zur Sterilisation das Einbringen beziehungsweise Einblasen des H₂O₂-Sterilisationsmediums und/oder der heißen sterilen Luft bzw. des Aktivierungsmediums über entsprechende Behandlungs- oder Sterilisationsköpfe erfolgt, die jeweils über ein Auslass- bzw. Abgaberohr zur Abgabe des H₂O₂-Sterilisationsmediums verfügen und beispielsweise auch als Gaslanzen bezeichnet werden. Insbesondere bei Linearmaschinen, wie Linearfüllern werden in einem Arbeitstakt mehrere Behälter gleichzeitig behandelt, wobei mehrere linear in Reihe angeordnete Gaslanzen eine Einheit, insbesondere eine Behandlungseinheit oder -einrichtung bilden und das H₂O₂-Sterilisationsmedium in mehrere, ebenfalls linear in Reihe angeordnete Behälter einblasen. Bei solchen, bekannten Linearmaschinen, insbesondere Linearfüllern, welche in der Regel taktweise betrieben sind bzw. im Schrittbetrieb laufen, werden die Behälter beispielsweise über so genannte Lineartransporteure transportiert, wobei derartige Lineartransporteure insbesondere auch Bestandteil der entsprechenden Behälterbehandlungsmaschinen sind.

Mittels derartiger, dem Fachmann bekannten, vorgenannten Lineartransporteure, werden die Behälter für ihre Behandlung an verschiedene in Transportrichtung auf einander folgenden Behandlungsstationen oder -positionen getaktet, d.h. schrittweise entlang der Transportstrecke bewegt, und zwar vorzugsweise in Form von Behältergruppen, welche eine Vielzahl von in einer linearen Reihe beabstandet zueinander angeordnete Behälter aufweist, die vorzugsweise an jeweils einem Behälterflansch hängend gehalten werden. Die Behälter einer Behältergruppe sind dabei relativ zueinander so angeordnet, dass Behälterlängsachsen der Behälter vorzugsweise parallel zueinander verlaufen und jeweils denselben Abstand aufweisen. Dabei ist es ebenfalls bekannt, dass die Behälter mittels einer linearen Aufnahmevorrichtung bzw. Behälterträgers, vorzugsweise mittels einer Trägerleiste mit entsprechenden Behälteraufnahmen hängend gehalten werden, wobei die eingehängten Behälter mit ihrem an die Füllöffnung angrenzenden Halsteil über die Trägerleiste nach oben vorstehen. Lineartransporteure mit Behälterträgern der genannten Art sind beispielsweise aus der WO 2013/004324 A1 bekannt.

Bei den bekannten Vorrichtungen und Verfahren werden die hängend in der Trägerleiste gehaltenen Behälter als Behältergruppe in eine entsprechende Sterilisierungsstation beziehungsweise in eine Vorrichtung zum Sterilisieren hinein gefördert und unterhalb der Behandlungseinrichtung, insbesondere unterhalb der Gaslanzen positioniert, so dass die Abgabe des Sterilisationsmediums in die Behälter, nämlich in den Behälterinnenraum im Taktbetrieb erfolgen kann. Bekannt ist dabei auch, dass im Bereich oberhalb der Trägerleiste des Lineartransporteurs ein aseptischer oder steriler Raum bzw. Aseptikraum, welcher auch als Aseptikzone bezeichnet wird, ausgebildet ist, so dass sich die die Füllöffnung bzw. die Behältermündung aufweisenden, nach oben über die Trägerleiste vorstehenden Teile der Behälter im Aseptikraum befinden, ebenso wie die eine jeweilige Auslassöffnung aufweisenden Abschnitte der Auslass- bzw. Abgaberohre. Zur Sterilisierung wird dann bei bekannten Vorrichtungen zunächst die Behandlungseinrichtung mit sämtlichen Gaslanzen derart mittels einer Senk-Bewegung nach unten in eine Betriebsstellung bewegt, dass die die Auslassöffnung aufweisenden Abschnitte der Auslass- bzw. Abgaberohre in die Behälter eintauchen. Schließlich wird der Ausstrom des H₂O₂-Sterilisationsmediums bewirkt, so dass das H₂O₂-Sterilisationsmedium ins Innere der Behälter eingeblasen wird.

Bei den bekannten linearen, im Schrittbetrieb betriebenen Sterilisationsvorrichtungen wird pro Arbeitstakt stets die gesamte Behältergruppe behandelt, das heißt, es werden pro Arbeitstakt alle Behandlungsköpfe bzw. Gaslanzen als Gruppe in die Betriebsstellung gebracht. Dabei besteht das Problem, dass im Falle eines fehlenden Behälters, d.h. wenn an einer der Behälteraufnahmen der Trägerleiste kein Behälter vorhanden ist, das Auslass- bzw. Abgaberohr in den unsterilen Raum unterhalb der Trägerleiste eingeführt wird und dadurch selbst einer Kontaminationsgefahr ausgesetzt ist.

Bei der aseptischen Abfüllung kommt jedoch der Aufrechterhaltung der Aseptikzone eine zentrale Bedeutung zu, weshalb gemäß den aus dem Stand der Technik bekannten Lösungen bisher versucht wird, eine mögliche Kontamination dadurch zu vermeiden, bei einem fehlenden Behälter in der Trägerleiste die gesamte Behandlungseinheit bzw. Behandlungseinrichtung mit allen Gaslanzen in der oberen Ausgangsstellung zu belassen und nicht in die Betriebsstellung abzusenken, um eine mögliche Kontamination sicher zu vermeiden. Dies bringt aber den erheblichen Nachteil mit sich, dass die vorhandenen, anwesenden Behälter nicht sterilisiert werden können und als Ausschussware entsorgt werden müssen. Daher besteht trotz der aus dem Stand der Technik bekannten Lösungen ein Bedarf an verbesserten Vorrichtungen zum Behandeln von Behältern mit Medium, insbesondere Sterilisierungsmedium.

Aufgabe der Erfindung ist es daher, eine Vorrichtung zum Behandeln von Behältern mit Medium, insbesondere Sterilisierungsmedium aufzuzeigen, die die Nachteile der aus dem Stand der Technik bekannten Lösungen behebt und die es ermöglicht, trotz konstruktiv einfachem Aufbau sowie unter sicherer Aufrechterhaltung der erforderlichen aseptischen Bedingungen die Sterilisierung der in der Vorrichtung für die Sterilisierung positionierten Behälter effektiv sicherzustellen.

Zur Lösung dieser Aufgabe ist eine Vorrichtung zum Behandeln von Behältern mit einem Medium entsprechend den Merkmalen des Patentanspruches 1 ausgebildet. Ferner ist ein Linearfüller zum aseptischen Befüllen von Behältern gemäß Anspruch 14 mit einer entsprechenden Vorrichtung ausgebildet und es ist ein Verfahren zum Behandeln von Behältern mit einem Medium mit den Merkmalen des Patentanspruches 15 angegeben. Vorteilhafte Weiterbildungen der Erfindung sind in den abhängigen Ansprüchen angegeben.

Die vorliegende Erfindung stellt eine Vorrichtung zum Behandeln von gruppenweise entlang eines Förderwegs im Taktbetrieb transportierten Behältern mit einem Medium, insbesondere einem gasförmigen, dampfförmigen oder flüssigen Medium bereit. Die Vorrichtung weist zumindest eine lineare Aufnahmevorrichtung mit mehreren Behälteraufnahmen zur hängenden Aufnahme der Behälter auf und umfasst wenigstens eine Behandlungsstation aufweisend zumindest eine Behandlungseinrichtung mit einer Zuleitung für das Medium sowie mit mehreren über jeweilige Zweigleitungen an die Zuleitung angeschlossenen Auslassköpfe. Wenigstens die Auslassköpfe sind mittels eines Antriebs gemeinsam als Gruppe in vertikaler Richtung bewegbar. Jeder Auslasskopf weist dabei eine vertikal orientierte Längsachse auf und umfasst eine sich entlang der Längsachse erstreckende Auslassleitung zur Abgabe des Mediums in die Behälter. Die Auslassleitung ist zur Behandlung des Behälters zumindest abschnittsweise in den in der Behälteraufnahme hängend aufgenommenen Behälter einführbar. Die Vorrichtung zeichnet sich insbesondere dadurch aus, dass jedem Auslasskopf wenigstens ein steuerbarer Rückhaltemechanismus zugeordnet ist, wobei die Vorrichtung ferner wenigstens eine mit dem Rückhaltemechanismus in kommunizierender Verbindung stehende Steuerung aufweist und wobei der steuerbare Rückhaltemechanismus zumindest ein Rückhalteelement aufweist und dazu ausgebildet ist, zumindest die Auslassleitung eines jeweiligen Auslasskopfes bedarfsgesteuert in einer Rückhalteposition zurückzuhalten.

"Behälter" sind im Sinne der Erfindung insbesondere solche, die unterhalb ihrer Behälteröffnung oder -mündung mit einem überstehenden Behälterflansch ausgebildet sind, z.B. Behälter oder Flaschen aus Kunststoff, beispielsweise aus PET.

Als "Medium" im Sinne der Erfindung sind sämtliche flüssigen, gasförmigen oder dampfförmigen Medien zu verstehen, die sich zur Behandlung von Behältern eignen. Beispielsweise kann es sich dabei um Sterilisierungsmittel bzw. -medien, Reinigungsmittel bzw. -medien, Trocknungsmittel bzw. -medien, Mittel bzw. Medien zum Beaufschlagen mit Inertgas oder Spanngas oder aber auch um Füllgut handeln.

Unter einem Transport "entlang eines Förderwegs im Taktbetrieb" ist dabei im Sinne der Erfindung insbesondere ein Transport zu verstehen, bei dem die zwischen einem Behältereinlauf und einem Behälterauslauf gebildete Transportstrecke zumindest in einem Teilbereich einen geradlinigen Verlauf besitzt, d.h. ein linearer Transport der Behälter. Der Transport erfolgt getaktet bzw. im Schrittbetrieb.

Die erfindungsgemäße Vorrichtung kann als Vorrichtung zum Behandeln der Behälter mit einem Sterilisierungsmedium verstanden werden und ist insbesondere als Vorrichtung zum Sterilisieren von Behältern zu verstehen, und zwar zum Sterilisieren mit einem Sterilisierungsmedium, insbesondere in bekannter Weise mit einem gasförmigen oder dampfförmigen bzw. aerosolartigen Sterilisierungsmedium, beispielsweise mit einem H₂O₂-haltigen Sterilisierungsmedium, welches vorliegend auch als H₂O₂-Aerosol bezeichnet werden kann. Das Sterilisieren mit H₂O₂-haltigem Sterilisierungsmedium umfasst in bekannter Weise auch die Verwendung von heißem Sterilisierungsmedium und/oder die Verwendung bzw. zusätzliche Verwendung von heißem Aktivierungsmedium und/oder heißer steriler Luft. Die Sterilisierung der Behälter ist vorliegend insbesondere als ein Behandlungsschritt zu verstehen, welcher einem Befüllen der Behälter mit flüssigem Füllgut unter aseptischen bzw. sterilen Bedingungen vorausgeht, wobei ein derartiges aseptisches Befüllen in einem Aseptikfüller, insbesondere in einem aseptischen Linearfüller erfolgt und die erfindungsgemäße Vorrichtung einer aseptischen Füllstation eines Aseptikfüllers vorgeschaltet oder auch als integrale Maschinenstation bzw. als integrales Bauteil eines aseptischen Linearfüllers ausgebildet sein kann. Es versteht sich von selbst, dass auch ein Trocknen der Behälter oder ein Gasaustreibungsschritt unter eine Behandlung der Behälter mit Medium im Sinne der Erfindung fällt.

Das Medium, insbesondere Sterilisierungsmedium wird über eine Zuleitung und entsprechende Zweigleitungen, welche Zweigleitungen auch als ein entsprechendes von der Zuleitung abzweigendes Anschlussverteilersystem verstanden werden kann, an mehrere, als Gruppe nebeneinander angeordnete Auslassköpfe der Behandlungseinrichtung zugeführt und über jeweilige Auslassleitungen, die wiederum mittels der Zweigleitungen mit der Zuleitung in Verbindung stehen, an die Behälter abgegeben und zur Behandlung, insbesondere Sterilisierung ins Innere der Behälter eingebracht. Die Abgabe des Sterilisierungsmediums kann vorliegend auch als Ausströmen des Sterilisierungsmediums aus der Auslassleitung und insbesondere als Einblasen des Sterilisierungsmediums in den Behälter verstanden werden, insbesondere auch als Beaufschlagen des Behälters mit Sterilisierungsmedium. Die Auslassköpfe mit der jeweiligen Auslassleitung können vorliegend als Lanze, Behandlungslanze bzw. Gas- oder Aerosollanze oder als Düse bzw. Aerosol-Düse oder als Gas- bzw. Aerosol-Verteiler oder -Auslass oder auch als Sprüheinheit bzw. Sprührohr bezeichnet werden.

Zumindest die Auslassköpfe der vorliegenden Vorrichtung sind mittels eines Antriebs in vertikaler Richtung bewegbar, wobei die vertikale Bewegung im Sinne der Erfindung auch als Hub-Senk-Bewegung oder als Auf- und Abbewegen zu verstehen ist und wobei vorzugsweise sämtliche Auslassköpfe der Behandlungseinrichtung als Gruppe gemeinsam über die Hub-Senk-Bewegung vertikal bewegt werden. Insbesondere werden die Auslassköpfe zusammen mit ihrer Zuleitung und ihren Zweigleitungen bzw. dem Anschlussverteilersystem auf- und abbewegt. Die Auslassköpfe können daher vorliegend auch als Hubelemente bezeichnet werden.

Im Sinne der vorliegenden Erfindung ist unter einer linearen Aufnahmevorrichtung mit mehreren Behälteraufnahmen insbesondere ein in bekannter Weise ausgeführter Behälterträger, insbesondere eine Übergabevorrichtung oder ein Transporteur zu verstehen, bei dem die Behälter mit einem unterhalb ihrer Behältermündung an einem Behälterhals ausgebildeten Behälterflansch während des Halterns bzw. Transportes hängend gehalten sind. Die lineare Aufnahmevorrichtung kann als zellenbrettartige, flache Trägerleiste ausgebildet sein, welche mit mehreren Behälteraufnahmen für ein Haltern der Behälter als Behältergruppe bzw. für einen mehrspurigen Transport der Behälter in Form einer Behältergruppe versehen ist, und zwar mit jeweils einer Behälteraufnahme für jede Spur des mehrspurigen Behälterstromes. Die Behälteraufnahmen der Trägerleiste können beispielsweise als Längsschlitz oder bevorzugt als Einzelöffnungen ausgebildet sein.

Die Behälter sind in der Aufnahmevorrichtung derart nebeneinander angeordnet bzw. eingehängt, dass jeweilige Behälterhauptachsen der in einer Aufnahmevorrichtung gehaltenen Behälter parallel zueinander orientiert sind. Zur Behandlung, insbesondere Sterilisierung werden die in den Behälteraufnahmen der Aufnahmevorrichtung eingehängten Behälter so unterhalb der Behandlungseinrichtung positioniert, dass jedem in der Aufnahmevorrichtung eingehängten Behälter ein Auslasskopf zugeordnet ist und die jeweiligen Längsachsen der Auslassköpfe mit den Behälterhauptachsen der jeweils zugeordneten Behälter in einer gemeinsamen vertikalen Achse zusammenfallen, welche auch als Behandlungsachse verstanden werden kann. Jeder Auslasskopf bildet in dieser Anordnung zusammen mit der zugeordneten Behälteraufnahme eine Behandlungsstelle, wobei ausgehend von einer Oberseite der Aufnahmevorrichtung oberhalb dieser ein Aseptikraum bzw. Sterilraum ausgebildet ist, in dem aseptische bzw. sterile Bedingungen herrschen. Der Aseptikraum wird vorliegend auch als Aseptikzone oder Sterilzone oder Hygienezone bezeichnet.

Erfindungsgemäß ist bei der vorliegenden Vorrichtung der steuerbare Rückhaltemechanismus jedem Auslasskopf zugeordnet, wobei über den Rückhaltemechanismus, vermittelt durch das Rückhalteelement, zumindest die Auslassleitung eines jeweiligen Auslasskopfes bedarfsgesteuert in einer Rückhalteposition zurückgehalten werden kann. Die Rückhalteposition ist dabei auch als eingezogene Position zu verstehen. Insbesondere ist dadurch bei der Vorrichtung sichergestellt, dass auch bei bzw. nach Absenkung der Auslassköpfe, insbesondere bei bzw. nach Absenkung der Behandlungseinrichtung, zumindest die Auslassleitung und dabei insbesondere deren unterer, die Auslassöffnung tragende Abschnitt, auf einem höher gelegenen Niveau gehalten wird, als in der nicht-zurückgehaltenen Stellung, insbesondere auch wenn an einer Behälteraufnahme der Aufnahmevorrichtung aus Fehlergründen kein Behälter vorhanden ist.

Insbesondere bevorzugt und ganz besonders vorteilhaft kann die gesteuerte Rückhaltung individuell und einzeln für jede Auslasseinheit erfolgen. Dies ist vorliegend auch als Rückhaltung, insbesondere Einzelrückhaltung oder Einzelarretierung zu verstehen. Insbesondere vorteilhaft kann dabei sichergestellt werden, dass bei einer zur Sterilisation durchgeführten Senkbewegung der Auslassköpfe bzw. der Behandlungseinrichtung, nämlich zum Beginnen des Sterilisationsvorganges, der die Auslassöffnung tragende Abschnitt der Auslassleitung, insbesondere auch einzelner, individueller Auslassleitungen stets im Aseptikraum bzw. Sterilraum angeordnet bleibt und nicht in den freien, insbesondere unsterilen Raum unterhalb der Aufnahmevorrichtung hineinbewegt wird, und zwar insbesondere auch wenn an einer Behälteraufnahme der Aufnahmevorrichtung aus Fehlergründen kein Behälter vorhanden ist. Die mit dem Rückhaltemechanismus in kommunizierender Verbindung stehende Steuerung ist vorzugsweise außerhalb der Aseptikzone angeordnet.

Darüber ist es besonders vorteilhaft möglich, auf Fehler an einzelnen Behandlungsstellen bzw. Behandlungspositionen, insbesondere auf das Fehlen von Behältern an einzelnen Behälteraufnahmen der Aufnahmevorrichtung zu reagieren. Vorteilhaft kann dadurch an einzelnen, unbelegten Behälteraufnahmen, an denen ein Behälter fehlt, die Auslassleitung zurückgehalten werden, und zwar derart, dass der freiendseitige Abschnitt der Auslassleitung vorzugsweise nicht in den Raum unterhalb der Aufnahmevorrichtung eintaucht. Vorteilhaft kann darüber die Prozesssicherheit erhöht werden, da die Auslassleitung beispielsweise die hygienische Zone, insbesondere die sterile bzw. aseptische Zone bei fehlendem Behälter nicht verlässt. Dadurch wird auch eine Rekontamination verhindert. Der Aseptikraum oberhalb der Aufnahmevorrichtung wird dabei vorteilhaft sicher aufrechterhalten.

Gleichzeitig kann dabei jedoch sichergestellt werden, dass die in der Aufnahmevorrichtung vorhandenen, gehalterten Behälter auf jeden Fall behandelt, vorzugsweise sterilisiert werden, und zwar an allen Behandlungspositionen, an denen ein Behälter vorhanden ist. Dies führt zu einer Leistungserhöhung durch deutlich effektivere Durchführung der Behandlung, insbesondere Sterilisierung, da Produktionsausschuss bei fehlenden Behältern vermieden wird, insbesondere bei Produktionsanfang und Produktionsende.

Ferner kann die Vorrichtung besonders vorteilhaft zur Optimierung des Behandlungsprozesses, insbesondere Sterilisationsprozesses und zu Testzwecken eingesetzt werden, da zum Beispiel die Auslassleitung einzeln gesteuert zurückgehalten werden kann, damit einzelne Schritte im Behandlungsprozess auch unterbrochen werden können. So können beispielsweise eine Behälterspur der Behältergruppe oder einzelne Behälter unterschiedlich behandelt werden, indem beispielsweise eine geringere Beaufschlagung mit Medium, insbesondere Sterilisationsmedium erfolgt und/oder eine Beaufschlagung mit Aktivierungsmedium und/oder heißer steriler Luft zum Trocknen individuell variiert oder unterbrochen bzw. weggelassen werden kann. Auch kann die Sicherheit erhöht werden, da es insbesondere möglich ist, die Auslasseinheiten durch Zurückhalten oder Einfahren stärker zurückzufahren und damit bei längeren Standzeiten eine Überhitzung der zu füllenden Behälter zu vermeiden.

Das Rückhalteelement ist vorzugsweise dazu eingerichtet, mit einem an jedem Auslasskopf vorgesehenen und mit der Auslassleitung in Wirkverbindung stehenden Kopplungsmittel gesteuert in Eingriff zu gelangen, wobei durch Eingriff des Rückhalteelementes in das Kopplungsmittel die Rückhaltung der Auslassleitung veranlasst wird.

Bevorzugt ist die Auslassleitung durch wenigstens zwei Teile, nämlich durch zumindest ein feststehendes Rohrstück und ein bewegliches Auslassendstück gebildet, wobei das Auslassendstück relativ zu dem Rohrstück in axialer Richtung entlang der Längsachse, insbesondere vertikal verschiebbar ist. Das Auslassendstück, welches an seinem freien Ende die Auslassöffnung umfasst, kann relativ zu dem Rohrstück eine zurückgehaltene und eine ausgefahrene Stellung einnehmen, insbesondere auf- und abgefahren, nämlich ausgefahren und eingezogen werden.

Dabei ist gemäß einer weiterhin vorteilhaften Ausführungsform die Auslasseinheit teleskopartig ausgebildet und das Auslassendstück ist koaxial zu dem Rohrstück angeordnet und axial verschiebbar zumindest teilweise in dem Rohrstück aufgenommen. Das Auslassendstück ist besonders bevorzugt über den Rückhaltemechanismus durch axiale Verschiebung in vertikaler Richtung nach oben von wenigstens einer ausgefahrenen Position in das Rohrstück einziehbar, und zwar in eine der Rückhalteposition entsprechende eingezogene Stellung, wobei eine Längserstreckung der Auslassleitung entlang der Längsachse in der ausgefahrenen Position größer ist als in der eingezogenen Rückhalteposition. Zur Rückhaltung der Auslassleitung in der Rückhalteposition erfolgt die axiale Verschiebung des Auslassendstücks in dem Rohrstück in gesteuerter Weise, und zwar vermittelt durch den Rückhaltemechanismus, wobei das Rückhalteelement vorzugsweise in ein mit dem Auslassendstück in Wirkverbindung stehendes Kopplungsmittel eingreift und dadurch das Auslassendstück zurückhält.

Besonders bevorzugt ist der Rückhaltemechanismus über ein Stellglied betätigbar, wobei das Stellglied gesteuert wird und beispielsweise rotativ oder linear wirken kann. Der Rückhaltemechanismus, insbesondere das Rückhalteelement kann beispielsweise gesteuert über das Stellglied eingedreht oder eingeschoben werden, und hält somit, vorzugsweise vermittelt über das Kopplungsmittel, die Auslassleitung, insbesondere das Auslassendstück vor dem Eintauchen in den Behälter oder in den freien, unsterilen Raum unterhalb der Aufnahmevorrichtung ab.

Gemäß einer weiterhin vorteilhaften Ausführungsvariante ist in jedem Auslasskopf ein inneres Ventil zur geregelten Abgabe des Mediums, insbesondere Sterilisierungsmediums vorgesehen, wobei das innere Ventil in einem geöffneten Zustand einen Abgabeströmungsweg für das Medium, insbesondere Sterilisierungsmedium durch die Auslassleitung freigibt und in einem geschlossenen Zustand den Abgabeströmungsweg verschließt und wobei das innere Ventil ganz besonders bevorzugt derart eingerichtet und ausgebildet ist, dass der Abgabeströmungsweg in der Rückhalteposition der Auslassleitung oder bei eingezogener Position des Auslassendstücks verschlossen ist. Das innere Ventil ist dabei vorzugsweise gesteuert.

Es versteht sich, dass das innere Ventil in alternativen Ausführungsformen auch derart ausgebildet sein kann, dass der Abgabeströmungsweg in der Rückhalteposition der Auslassleitung oder bei eingezogener Position des Auslassendstücks offen ist, und beispielsweise im Falle eines Sterilisierungsprozesses an einer unbelegten Behälteraufnahme das Sterilisierungsmedium in die Aseptikzone oder aber auch in den unsterilen Bereich unterhalb der Aufnahmevorrichtung einströmt, wodurch der die Aseptikzone aufrechterhaltende Effekt zusätzlich verstärkt werden kann.

Ebenso bevorzugt ist eine Ausführungsvariante, bei der in jedem Auslasskopf eine innere Drossel vorgesehen ist, wobei die innere Drossel beispielsweise derart eingerichtet und ausgebildet ist, dass in der Rückhalteposition der Auslassleitung oder bei eingezogener Position des Auslassendstücks die Abgabe des Mediums, insbesondere Sterilisierungsmediums über die Auslassleitung gedrosselt ist, derart, dass in der Rückhalteposition der Auslassleitung oder in der eingezogenen Position des Auslassendstücks ein Fluss des Mediums, insbesondere Sterilisierungsmediums aus der Auslassleitung vermindert ist. Die innere Drossel ist dabei vorzugsweise gesteuert. Darüber ist beispielsweise auch möglich im Falle eines Sterilisierungsprozesses an einer unbelegten Behälteraufnahme das Sterilisierungsmedium mit vermindertem Fluss bzw. verminderter Strömung in die Aseptikzone oder aber auch in den unsterilen Bereich unterhalb der Aufnahmevorrichtung einströmt. Dadurch kann der die Aseptikzone aufrechterhaltende Effekt bei sparsamerem Verbrauch von Sterilisierungsmedium zusätzlich verstärkt werden.

Erfindungsgemäß umfasst die Vorrichtung ferner zumindest eine sensorische Erfassungseinheit zur Erfassung der Anwesenheit von Behältern in den Behälteraufnahmen der Aufnahmevorrichtung, wobei die sensorische Erfassungseinheit in kommunizierender Verbindung mit der Steuerung steht. Insbesondere bevorzugt ist die sensorische Erfassungseinheit dabei durch wenigstens einen optischen Sensor oder durch einen Lagesensor gebildet. Als optische Sensoren können insbesondere auch elektronische Kameras, Lichtschranken oder dergleichen eingesetzt werden.

Insbesondere kann durch die sensorische Erfassungseinheit festgestellt werden, welche Behandlungspositionen belegt oder unbelegt sind. Die entsprechenden Messdaten bzw. Signale der mit der Steuerung zusammenwirkenden sensorischen Erfassungseinheit werden über eine entsprechende Übertragungs- und Auswerteeinrichtung an die Steuerung weitergegeben und ausgewertet. Die Steuerung gibt daraufhin Steuersignale an den ebenfalls mit der Steuerung zusammenwirkenden Rückhaltemechanismus bzw. entsprechende Stellglieder aus, um die Auslassleitung, insbesondere das Auslassendstück an jeweiligen unbelegten Behandlungspositionen zurückzuhalten.

Die Steuerung des Rückhaltemechanismus und des inneren Ventils oder inneren Drossel zur entsprechenden Regelung bzw. Einstellung der Abgabe des Sterilisierungsmediums erfolgt vorzugsweise in einer aufeinander abgestimmten Weise.

Gemäß einer weiterhin bevorzugten Ausführungsform der Erfindung sind die Auslassleitungen, insbesondere die Auslassleitungen mit den zugehörigen, verbundenen Zweigleitungen und der Zuleitung derart höhenverstellbar ausgebildet, dass die Auslassöffnungen auf wenigstens eine vorgegebene Höhe und/oder stufenweise auf verschiedene Höhen oberhalb der Aufnahmevorrichtung positionierbar sind.

Beispielsweise kann das Auslassendstück bei fehlendem Behälter in einer Behälteraufnahme der Aufnahmevorrichtung mittels der Steuerung und des Rückhaltemechanismus so auf einer Höhe positioniert werden, dass die Auslassöffnung in einem Bereich von etwa 0,5 mm bis 5 mm oberhalb der Oberseite der Aufnahmevorrichtung angeordnet ist. Gleichzeitig kann die Abgabe des Sterilisierungsmediums mittels der Steuerung so eingestellt werden, dass das Sterilisierungsmedium durch die Behälteraufnahme in den unsterilen Raum eingebracht wird, um die Aufrechterhaltung der aseptischen Bedingungen in der Aseptikzone zusätzlich zu sichern. Auch kann beispielsweise bei fehlendem Behälter der Volumenstrom für die Abgabe des Sterilisierungsmedium durch die Behälteraufnahme in den unsterilen Raum variiert, insbesondere verringert werden.

Besonders bevorzugt ist die Vorrichtung als integrale Maschinenstation eines aseptischen Linearfüllers ausgebildet. Die Vorrichtung kann insbesondere als Vorrichtung zum Sterilisieren von Behältern ausgebildet sein.

Die Erfindung stellt auch einen Linearfüller zum aseptischen Befüllen von Behältern mit einem flüssigen Füllgut bereit, welcher zumindest eine Transportvorrichtung für den Transport der Behälter in einer Transportrichtung und zumindest eine Füllstation aufweist. Der Linearfüller ist im Schrittbetrieb betrieben und weist eine wie oberhalb beschriebene, in Transportrichtung vor der Füllstation angeordnete und eine Behältersterilisierung des Linearfüllers bildende Vorrichtung zum Behandeln der Behälter mit einem Medium, insbesondere Sterilisierungsmedium auf.

Die Erfindung stellt auch ein Verfahren zum Behandeln von gruppenweise entlang eines Förderwegs im Taktbetrieb transportierten Behältern mit einem Medium, insbesondere Sterilisierungsmedium bereit. Bei dem Verfahren werden mittels einer linearen Aufnahmevorrichtung mit mehreren Behälteraufnahmen die Behälter in Form einer geordneten Behältergruppe mit vorgegebener Anzahl an Behältern hängend gehalten und in einer Behandlungsstation einer Vorrichtung unterhalb einer Behandlungseinrichtung positioniert. Die Behandlungseinrichtung umfasst eine Zuleitung für das Medium und mehrere über Zweigleitungen an die Zuleitung angeschlossene Auslassköpfe. Jedem Behälter der Behältergruppe ist dabei ein Auslasskopf zugeordnet, wobei bei dem Verfahren die Auslassköpfe als Gruppe gemeinsam mittels eines entsprechenden Antriebs durch eine Hub-Senkbewegung in vertikaler Richtung nach unten bewegt und dadurch den zugeordneten Behältern derart angenähert werden, dass eine bei jedem Auslasskopf vorgesehene Auslassleitung für die Abgabe des Mediums über eine jeweilige Behältermündung wenigstens teilweise in den zugeordneten Behälter eingeführt wird. Das Verfahren zeichnet sich insbesondere dadurch aus, dass die Auslassleitung eines jeweiligen Auslasskopfes mittels eines durch eine Steuerung gesteuerten Rückhaltemechanismus bedarfsgesteuert in einer Rückhalteposition zurückgehalten wird.

Bei Vorhandensein eines Behälters an einer jeweiligen Behälteraufnahme ist die Auslassleitung im abgesenkten Zustand der Auslassköpfe, insbesondere bei abgesenkter Behandlungseinrichtung, derart angeordnet, dass diese in einer ausgefahrenen Position verbleibt und zur Abgabe des Sterilisierungsmediums wenigstens teilweise in den zugeordneten Behälter eingeführt wird.

Besonders bevorzugt kann die Auslassleitung jedes einzelnen Auslasskopfes bedarfsgesteuert und individuell zurückgehalten werden, insbesondere bevorzugt dort, wo ein Behälter in der Behältergruppe fehlt und daher die dem Auslasskopf zugeordnete Behälteraufnahme der linearen Aufnahmevorrichtung unbelegt ist.

Vorzugsweise handelt es sich um ein Verfahren zum Sterilisieren von Behältern, wobei zumindest in der Behandlungsstation der Vorrichtung wenigstens in einem Bereich oberhalb der Aufnahmevorrichtung eine Aseptikzone ausgebildet und aufrechterhalten wird, und zwar zumindest in dem Bereich, in dem die Behältermündungen der hängend gehaltenen Behälter angeordnet sind. Die Auslassleitung wird bedarfsgesteuert derart zurückgehalten, dass die zurückgehaltene Auslassleitung in der Rückhalteposition vollständig in der Aseptikzone angeordnet ist.

Weiterhin bevorzugt umfasst die Auslassleitung zumindest ein feststehendes Rohrstück und ein bewegliches Auslassendstück, wobei das Auslassendstück zum Einnehmen der Rückhalteposition der Auslassleitung in axialer Richtung entlang der Längsachse des jeweiligen Auslasskopfes relativ zu dem Rohrstück verschoben, insbesondere in das Rohrstück eingezogen wird.

Die Erfindung wird im Folgenden anhand der Figuren an Ausführungsbeispielen näher erläutert. Es zeigen:
- Fig. 1a: in sehr vereinfachter schematischer Ansicht eine Ausführungsform einer Vorrichtung zum Behandeln von Behältern mit Sterilisierungsmedium, dargestellt mit der Behandlungseinrichtung in einer ersten, oberen Höhenlage;
- Fig. 1b: die Ansicht gemäß Figur 1a, dargestellt mit der Behandlungseinrichtung in einer zweiten, abgesenkten Höhenlage im Normalbetrieb;
- Fig. 1c: die Ansicht gemäß Figur 1b, dargestellt im Betrieb bei fehlendem Behälter an einer Behandlungsposition;
- Fig. 2A-C: in sehr vereinfachten Teildarstellungen ausschnittsweise eine Ausführungsform der Vorrichtung in jeweils verschiedenen Betriebszuständen,
- Fig. 3a: einen Schnitt durch eine Ausführungsform der Behandlungseinrichtung,
- Fig. 3b: im Schnitt eine Teildarstellung der Auslasseinheit der Behandlungskopfeinheit aus Figur 3a und
- Fig. 4A-C: einen Schnitt durch eine Ausführungsform der Behandlungseinrichtung und im Schnitt Teildarstellungen davon,
- Fig. 5: eine stark vereinfachte schematische Darstellung eines aseptischen Linearfüllers.

Die in den Figuren allgemein mit 1 bezeichnete Vorrichtung zum Behandeln von gruppenweise entlang eines Förderwegs transportieren Behältern 2 mit einem Medium, insbesondere Sterilisierungsmedium dient insbesondere zur Sterilisierung der Behälter 2, insbesondere von PET-Flaschen mit einem H₂O₂-haltigen Sterilisationsmedium und kann vorliegend auch als Vorrichtung zum Sterilisieren bezeichnet werden.

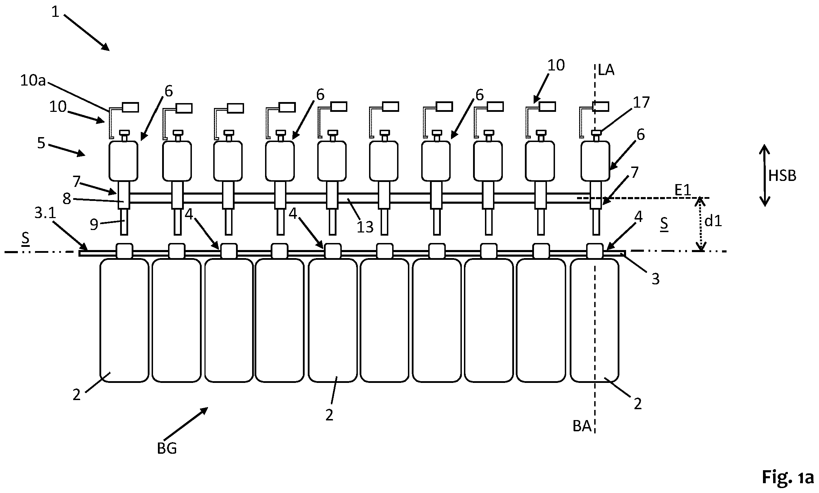

Die Vorrichtung 1 umfasst eine in bekannter Weise ausgeführte lineare Aufnahmevorrichtung 3 mit mehreren Behälteraufnahmen 4 zur hängenden Aufnahme der Behälter 2, wobei die Behälter 2 mittels der Aufnahmevorrichtung 3 als Behältergruppe BG gehalten, entsprechend in der Vorrichtung 1 positioniert und auch getaktet in parallelen Linien weitertransportiert werden. In der in Figuren 1a bis 1c beispielhaft dargestellten Ausführungsform ist die Aufnahmevorrichtung 3 mit zehn Behälteraufnahmen 4 ausgestattet, so dass die Anzahl der gleichzeitig gehalterten und transportierten Behälter 2 einer Behältergruppe BG gleich zehn ist. Die Behälterzahl kann vorliegend auch als Gruppenanzahl oder Gruppengröße verstanden werden und entspricht dabei insbesondere gleichzeitig der Anzahl bzw. Zahl der in der Vorrichtung vorhandenen Behandlungsstellen oder Behandlungspositionen.

Die eingehängten Behälter 2 sind in einer linearen Reihe beabstandet derart zueinander angeordnet, dass jeweilige Behälterhauptachsen BA der Behälter 2 einer Behältergruppe BG vorzugsweise parallel zueinander verlaufen und jeweils denselben Abstand aufweisen. Die Behälter 2 stehen mit ihrem an die Füllöffnung angrenzenden Halsteil über eine Oberseite 3.1 der Aufnahmevorrichtung 3 nach oben vor, wobei der Raum oberhalb der Oberseite 3.1 der Aufnahmevorrichtung 3 als Aseptikraum S bzw. Aseptikzone ausgebildet ist, in dem sterile bzw. aseptische Bedingungen herrschen.

Die Vorrichtung 1 umfasst ferner eine Behandlungseinrichtung 5 mit mehreren, ebenfalls in einer linearen Reihe beabstandet zueinander angeordneten Auslassköpfen 6, welche im dargestellten Beispiel ebenfalls in einer Anzahl von zehn vorgesehen sind. Die Auslassköpfe 6 weisen jeweilige vertikal orientierte Längsachsen LA auf, die wiederum parallel zueinander verlaufen. Jeder Auslasskopf 6 ist einer Behälteraufnahme 4 der Aufnahmevorrichtung 3 zugeordnet und bildet zusammen mit dieser jeweils eine Behandlungsstelle oder Behandlungsposition. Die jeweiligen Behälterhauptachsen BA der in den Behälteraufnahmen 4 hängenden und zur Sterilisierung positionierten Behälter 2 fallen dabei mit den jeweiligen Längsachsen LA der zugeordneten Behandlungskopfeinheiten 6 zusammen und bilden eine gemeinsame Behandlungsachse.

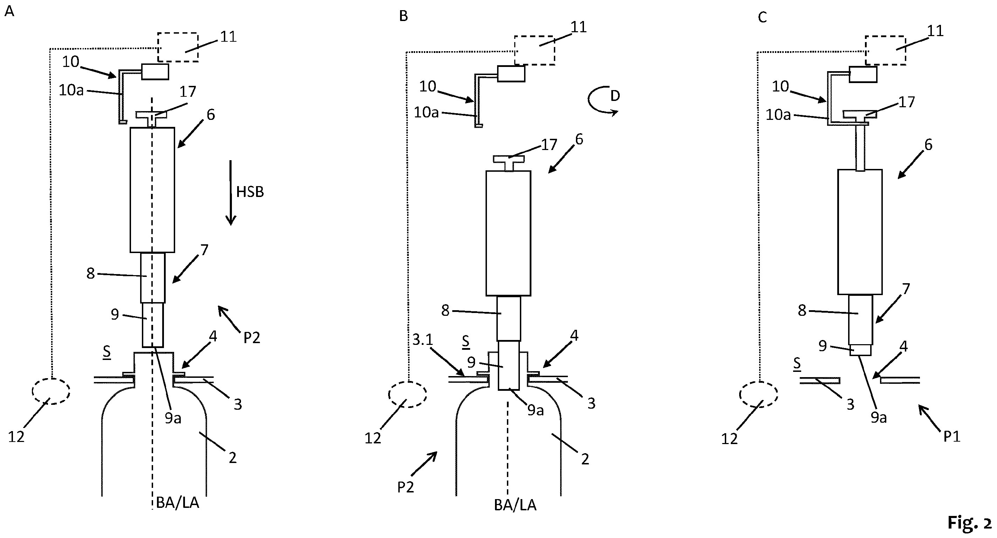

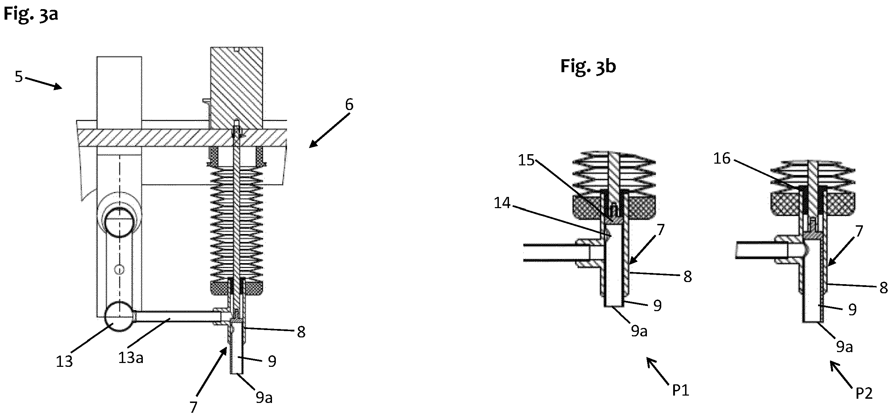

Die Behandlungseinrichtung 5 umfasst ferner eine Zuleitung 13 für das Sterilisationsmedium, wobei die Auslassköpfe 6 über entsprechende Zweigleitungen 13a (siehe Figur 3a, 3b) bzw. ein Anschlussverteilersystem an die Zuleitung 13 angeschlossen sind. Jeder Auslasskopf 6 umfasst eine längliche, sich entlang der Längsachse LA erstreckende Auslassleitung 7 zur Abgabe des Sterilisationsmediums in die Behälter 2. Die Auslassleitung 7 weist im bevorzugten dargestellten Beispiel der Figuren 1a bis 1c ein feststehendes Rohrstück 8 und ein bewegliches Auslassendstück 9 auf, welches an seinem freien Ende eine Auslassöffnung 9a (siehe Figur 2) umfasst. Das Auslassendstück 9 ist relativ zu dem feststehenden Rohrstück 8 axial entlang der Längsachse LA verschiebbar und ist im dargestellten Beispiel teilweise in dem Rohrstück 8 aufgenommen, so dass die Auslassleitung 7 teleskopartig ausgebildet ist.

Die Auslassköpfe 6 des dargestellten Beispiels sind zusammen mit ihrem Anschlussverteilersystem 13a und der Zuleitung 13 in vertikaler Richtung mittels einer Hub-Senk-Bewegung HSB bewegbar, so dass im Beispiel der Figuren 1a bis 1c die Behandlungseinrichtung 5 insgesamt hub- und senkbeweglich ausgebildet ist. Die Hub-Senk-Bewegung erfolgt durch einen nicht in den Figuren dargestellten Antrieb. Die Behandlungseinrichtung 5 kann aufgrund der Hub-Senk-Bewegung HSB von einer ersten, oberen Höhenlage in eine zweite abgesenkte Höhenlage bewegt werden und umgekehrt, wobei die erste, obere Höhenlage eine Transportstellung darstellt, in der die Behälter 2 während eines Taktes transportiert werden können und wobei die zweite abgesenkte Höhenlage eine Behandlungsstellung darstellt, in der die Behälter 2 mit dem Medium behandelt, insbesondere beaufschlagt werden.

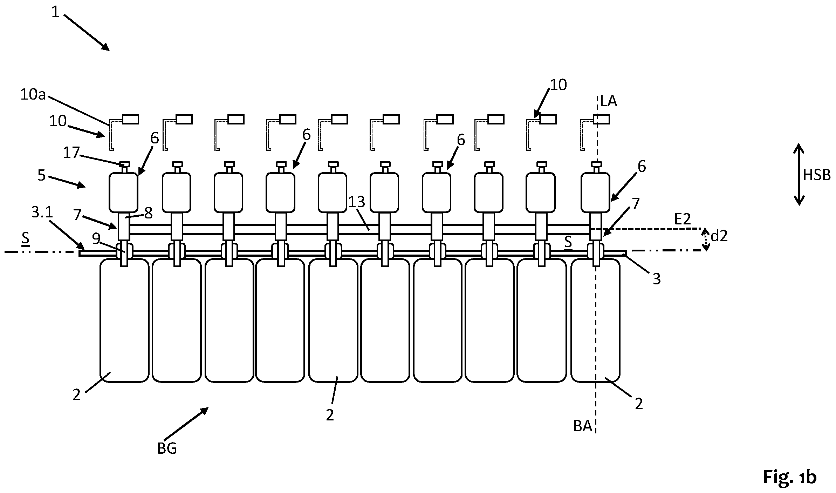

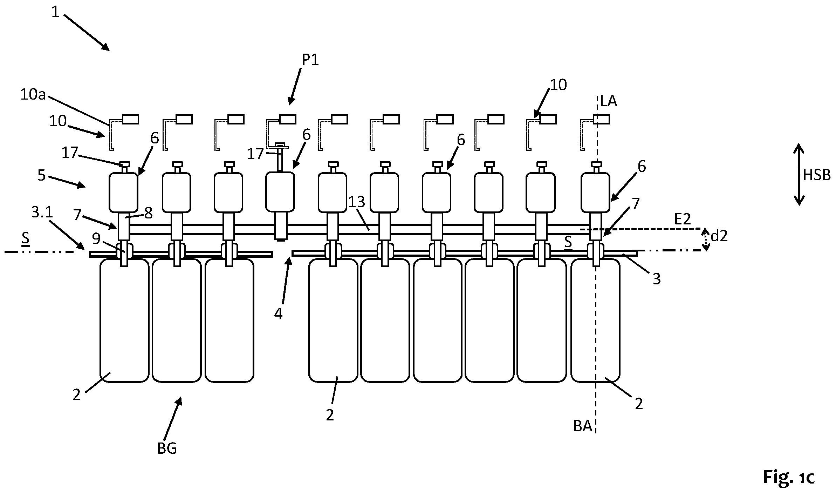

In der ersten, oberen Höhenlage nimmt die Behandlungseinrichtung 5 eine Lage ein, in der im dargestellten Beispiel die Zuleitung 13 auf einem ersten Höhenniveau in einer ersten Niveauebene E1 zu liegen kommt, welche in einem ersten Höhenabstand d1 zur Oberseite 3.1 der Aufnahmevorrichtung 3 verläuft, wie in Figur 1a dargestellt. In der zweiten, abgesenkten Höhenlage nimmt die Behandlungseinrichtung 5 eine Lage ein, in der im dargestellten Beispiel die Zuleitung 13 auf einem zweiten Höhenniveau in einer zweiten Niveauebene E2 zu liegen kommt, welche in einem geringeren, zweiten Höhenabstand d2 zur Oberseite 3.1 der Aufnahmevorrichtung 3 verläuft, wie in Figuren 1b und 1c dargestellt. Figur 1b zeigt dabei einen Normalbetrieb, wenn sämtliche Behälteraufnahmen 4 der Aufnahmevorrichtung 3 mit je einem Behälter 2 belegt bzw. besetzt sind. Figur 1c zeigt die Vorrichtung im Behandllungsbetrieb, wenn an einer Behälteraufnahme 4, in Figur 1c an der Behandlungsposition "vier", nämlich an vierter Stelle von links, der Behälter 2 fehlt und die entsprechende Behälteraufnahme 4 daher unbelegt ist.

In der beispielhaft dargestellten Ausführungsform der Figuren 1a bis 1c ist für jeden Auslasskopf 6 ferner ein mittels einer Steuerung 11 (siehe Figur 2) gesteuerter Rückhaltemechanismus 10 mit einem Rückhalteelement 10a zum Rückhalten der Auslassleitung 7, insbesondere des Auslassendstücks 9 in einer Rückhalteposition P1 vorgesehen, wobei die Rückhalteposition P1 bei der dargestellten Ausführungsform auch als eingezogene Position verstanden werden kann.

In Figur 1c ist an Behandlungsposition "vier" aufgrund des dort fehlenden Behälters 2 die Auslassleitung 7 entsprechend in der Rückhalteposition P1 angeordnet. Gesteuert über die Steuerung 11 (siehe Figur 2) wird das Rückhaltelement 10a dazu so angesteuert bzw. aktiviert, dass es in ein am Auslasskopf 6 vorgesehenes Kopplungsmittel 17 eingreift, welches in Wirkverbindung mit der Auslassleitung 7 steht, so dass die Auslassleitung 7 vermittelt über das in Eingriff mit dem Rückhalteelement 10a stehende Kopplungsmittel 17 zurückgehalten wird und dadurch auch in der zweiten, abgesenkten Höhenlage der Behandlungseinrichtung 5 nicht in den unsterilen Raum unterhalb der Aufnahmevorrichtung 3 eintaucht, sondern vielmehr gänzlich innerhalb der Aseptikzone S gehalten wird.

Mit Bezug auf die Figur 2, Darstellungen A bis C sind mehr im Detail einzelne Betriebszustände beziehungsweise Positionen der Behandlungseinrichtung 5, insbesondere der Auslassleitung 7 der Auslassköpfe 6 gezeigt.

Abschnitt A der Figur 2 zeigt einen Zustand an einer mit einem Behälter 2 belegten Behälteraufnahme 4, bei dem die Behandlungseinrichtung 5 die erste, obere Höhenlage einnimmt, d.h. noch nicht im Arbeitstakt mittels Senkbewegung HSB abgesenkt bzw. nach unten bewegt wurde. Dies stellt quasi eine Ausgangsstellung vor bzw. zu Beginn des Sterilisationsprozesses dar. Das Auslassendstück 9 befindet sich in ausgefahrener Position P2.

Abschnitt B der Figur 2 zeigt den Zustand an der mit einem Behälter 2 belegten Behälteraufnahme 4, bei dem die Behandlungseinrichtung 5 im Arbeitstakt bereits mittels Senkbewegung HSB abgesenkt bzw. nach unten bewegt wurde und die zweite abgesenkte Höhenlage einnimmt. Dies stellt quasi eine Behandlungsstellung während des Sterilisationsprozesses dar. Das Auslassendstück 9 befindet sich auch hier in der zweiten ausgefahrenen Position P2, so dass die Längserstreckung der Auslassleitung 7 deutlich größer ist als die axiale Länge des Rohrstücks 8. Das Auslassendstück 9 ist dabei über die Behältermündung in den Behälter 2 eingeführt. Die Abgabe des Sterilisationsmediums über die Auslassöffnung 9a erfolgt gesteuert, indem mittels der Steuerung 11 eine Öffnung eines inneren Ventils in dem Behandlungskopf 6 vermittelt wird. Der Rückhaltemechanismus 10 greift nicht, da über die sensorische Erfassungseinheit 12 das Vorhandensein eines Behälters 2 an der Behandlungsstelle detektiert wird.

Abschnitt C der Figur 2 zeigt den Zustand an einer unbelegten Behälteraufnahme 4, bei der über die sensorische Erfassungseinheit 12 das Fehlen eines Behälters 2 an der Behandlungsstelle detektiert wird. Hierbei greift, vermittelt über die Steuerung 11, der Rückhaltemechanismus 10, so dass das Auslassendstück 9, trotz bereits abgesenkter bzw. nach unten bewegter Behandlungseinrichtung 5, in der Rückhalteposition P1 gesteuert zurückgehalten wird. Die Auslassöffnung 9a des Auslassendstücks 9 ist dabei nach wie vor innerhalb des Aseptikraumes S angeordnet, so dass eine Kontamination des Auslassendstücks 9 vermieden wird. In dieser Position ist es je nach Ausführungsform und je nach Anwendungsfall möglich die Abgabe des Sterilisationsmediums über die Auslassöffnung 9a gesteuert einzustellen, und zwar derart, dass eine Abgabe des Sterilisationsmediums in den Aseptikraum S oder in den unsterilen Raum unterhalb der Aufnahmevorrichtung 3 bei vollem oder gedrosseltem Volumenstrom erfolgt oder dass keine Abgabe erfolgt.

In dem dargestellten Beispiel der Figur 2 kann das Rückhalterelement 10a des Rückhaltemechanismus 10 über ein Stellglied rotativ durch eine Drehbewegung D so eingedreht werden, dass es in Eingriff mit dem Kopplungsmittel 17 gelangt, welches wiederum in Wirkverbindung mit der Auslassleitung, insbesondere mit dem Auslassendstück 9 steht, so dass beim Absenken der Behandlungseinrichtung 5 durch die Hub-Senk-Bewegung HSB die axiale Verschiebung des Auslassendstücks 9 in das Rohrstück 8 hinein bewirkt wird und das Auslassendstück 9 in der eingezogenen Position P1 zurückgehalten wird. Alternativ ist jedoch auch denkbar, dass der Rückhaltemechanismus 10 über eine lineare Bewegung greift.

Den in den Figuren dargestellten vorstehend beschriebene Beispielen ist gemein, dass die Auslassleitung 7 zweiteilig, teleskopartig ausgebildet ist und die Rückhalteposition P1 durch axiale Verschiebung des Auslassendstücks 9 in dem Rohrstück 8 eingenommen werden kann. Es versteht sich, dass vorliegend selbstverständlich auch nicht in den Figuren dargestellte, alternative Ausführungsformen umfasst sind. Beispielsweise könnte die Auslassleitung 7 faltenbalgartig ausgebildet sein, wobei gesteuert eine gewisse Stauchung der faltenbalgartigen Auslassleitung 7 vermittelt wird, und zwar durch Eingriff des Rückhalteelementes 10a in das mit der faltenbalgartigen Auslassleitung 7 in Wirkverbindung stehende Kopplungsmittel 17. Auch ist es denkbar, die gesamte Auslassleitung 7 in den Auslasskopf 6 zurückzuziehen. Ebenfalls kann, sofern eine flexible Zuleitung 13 und eine flexibles Anschlussverteilersystem 13a dies zulassen, der gesamte Auslasskopf 6 zusammen mit der Auslassleitung 7 in einer höheren Höhenlage zurückgehalten werden.

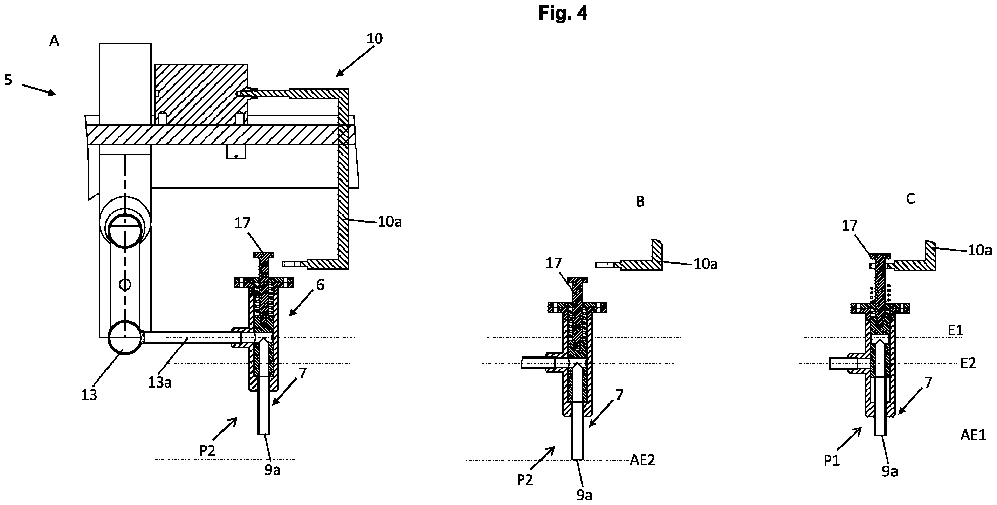

Die Figuren 3a und 3b sowie die Figur 4 zeigen als Teilausschnitt jeweils im Schnitt weitere Ausführungsvarianten der Vorrichtung 1, insbesondere den Bereich des Auslasskopfes 6 mit Auslassleitung 7und Rückhaltemechanismus 10. Bei der dargestellten Variante der Figuren 3a und 3b erfolgt das Verschieben des Auslassendstücks 9 relativ zum Rohrstück 8 gesteuert über einen Stellmotor, welcher außerhalb der Aseptikzone S angeordnet ist und einen Zylinder 15 bzw. Stempel betätigt, der mit dem Auslassendstück 9 verbunden ist. Über die Betätigung des Zylinders 15 kann das Auslassendstück 9 in vertikaler Richtung ein- bzw. ausgefahren werden. In diesem Fall dient der Zylinder 15 gleichzeitig als Kopplungsmittel für die Zusammenwirkung mit dem Rückhaltemechanismus 10. In dieser Ausführungsvariante dient das Auslassendstück 9 somit auch als Stellmittel für das innere Ventil.

Das Auslassendstück 9 weist einen Durchlass 14 auf, welcher in der ausgefahrenen Position P2 gemäß Figur 3b derart angeordnet ist, dass ein Abgabeströmungsweg für das Sterilisationsmedium vom Anschlussverteilersystem 13a über die Auslassleitung 7 freigegeben wird. In der eingezogenen Position P1 ist der Abgabeströmungsweg verschlossen. Zusätzlich sind Dichtungen 16 vorgesehen, die den Abgabeströmungsweg abdichten.

Wie aus Figur 3a hervorgeht, kann bei der dargestellten Variante auch bei einer noch weiter ausgefahrenen Position des Auslassendstücks 9 der Abgabeströmungsweg wieder verschlossen werden, indem der Durchlass 14 auf eine weitere schließende Stellung gebracht wird.

Aus der Figur 4 ist noch einmal im Detail das Rückhalten der Auslassleitung 7 dargestellt. In Abschnitt A der Figur 4 ist die Behandlungseinrichtung 5 in der ersten, höheren Höhenlage angeordnet und die Zuleitung 13 kommt auf dem ersten Höhenniveau in der ersten Niveauebene E1 zu liegen. Dabei ist die Auslassöffnung 9a der Auslassleitung 7, welche in ausgefahrener Position P2 befindlich ist, in einer ersten Auslassebene AE1 aufgenommen. In den Abschnitten B und C ist die Behandlungseinrichtung 5 in der zweiten, abgesenkten Höhenlage angeordnet und die Zuleitung 13 kommt auf dem zweiten Höhenniveau in der zweiten Niveauebene E2 zu liegen. Abschnitt B zeigt dabei die ausgefahrene Position P2 der Auslassleitung 7, so dass die Auslassöffnung 9a in der abgesenkten Höhenlage der Behandlungseinrichtung 5 in einer tiefer gelegenen, zweiten Auslassebene AE2 aufgenommen ist. Abschnitt C zeigt im Gegensatz dazu die Rückhalteposition P1 der Auslassleitung 7, bei der die Auslassöffnung 9a auch in der abgesenkten Höhenlage der Behandlungseinrichtung 5 in der ersten Auslassebene AE1 aufgenommen ist.

Die Rückhaltung erfolgt mittels des Rückhalteelementes 10a des Rückhaltemechanismus 10, welches gesteuert in Eingriff mit dem Kopplungsmittel 17 gelangt. Das Kopplungsmittel 17 ist im dargestellten Beispiel stempelartig ausgebildet und mit der Auslassleitung 7 verbunden. Durch entsprechenden Eingriff des Rückhalteelementes 10a in das Kopplungsmittel 17 vor bzw. beim Absenken der Behandlungseinrichtung 5 kann eine Auslassleitung 7 eines entsprechenden Auslasskopfes 6 oder können die Auslassleitungen 7 mehrerer Auslassköpfe 6 individuell, bedarfsgesteuert zurückgehalten werden. Bei dem in Figur 4 dargestellten Beispiel ist der Abgabeströmungsweg in der ausgefahrenen Position P2 freigegeben und offen (Abschnitt a und B) und in der Rückhalteposition P1 verschlossen (Abschnitt C). Es versteht sich von selbst, dass alternativ auch auch andere Steuerungs- und oder Schaltungsvarianten für den Abgabeströmungsweg möglich sind.

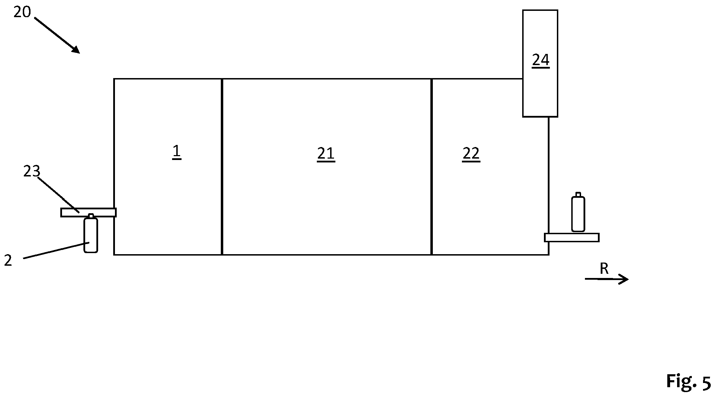

Figur 5 zeigt ganz grob schematisch einen im Schrittbetrieb betriebenen Linearfüller 20 zum aseptischen Befüllen von Behältern 2 mit einem flüssigen Füllgut. Der Linearfüller 20 weist eine Füllstation 21 und eine Transportvorrichtung 23 für den getakteten Transport der Behälter 2 in einer Transportrichtung R auf. In Transportrichtung R vor der Füllstation 21 ist eine Vorrichtung 1 zum Sterilisieren der Behälter 2 vorgesehen, welche als Teil des Linearfüllers 20 eine von diesem umfasste Behältersterilisierungsstation bildet. Der Linearfüller weist in Transportrichtung R nach der Füllstation 21 ferner eine Verschließstation 22 auf, welcher über eine Behälterverschlusszufuhr 24 sterilisierte Behälterverschlüsse zugeführt werden und welche die unter aseptischen Bedingungen befüllten Behälter 2 unter ebenfalls aseptischen Bedingungen verschließt.

### Bezugszeichenliste

- 1: Vorrichtung zum Behandeln von Behältern
- 2: Behälter
- 3: lineare Aufnahmevorrichtung
- 3.1: Oberseite
- 4: Behälteraufnahmen
- 5: Behandlungseinrichtung
- 6: Auslasskopf
- 7: Auslassleitung
- 8: Rohrstück
- 9: Auslassendstück
- 9a: Auslassöffnung
- 10: Rückhaltemechanismus
- 10a: Rückhalteelement
- 11: Steuerung
- 12: sensorische Erfassungseinheit
- 13: Zuleitung
- 13a: Zweigleitungen bzw. Anschlussverteilersystem
- 14: Durchlass
- 15: Zylinder
- 16: Dichtung
- 17: Kopplungsmittel
- 20: Linearfüller
- 21: Füllstation
- 22: Verschließstation
- 23: Transportvorrichtung
- 24: Behälterverschlusszufuhr

- AE1: erste Auslassebene
- AE2: zweite Auslassebene
- BA: Behälterhauptachse

- BG: Behältergruppe
- D: Drehbewegung
- d1: erster Höhenabstand
- d2: zweiter Höhenabstand
- E1: erste Niveauebene
- E2: zweite Niveauebene
- HSB: Hub-Senk-Bewegung
- LA: Längsachse
- P1: eingezogene Position
- P2: ausgefahrene Position
- S: Aseptikraum

## Patentansprüche

1. Vorrichtung (1) zum Behandeln von gruppenweise entlang eines Förderwegs im Taktbetrieb transportierten Behältern (2) mit einem Medium, insbesondere einem gasförmigen, dampfförmigen oder flüssigen Medium, aufweisend zumindest eine lineare Aufnahmevorrichtung (3) mit mehreren Behälteraufnahmen (4) zur hängenden Aufnahme der Behälter (2), wenigstens eine Behandlungsstation umfassend zumindest eine Behandlungseinrichtung (5) mit einer Zuleitung (13) für das Medium sowie mit mehreren über jeweilige Zweigleitungen (13a) an die Zuleitung (13) angeschlossenen Auslassköpfen (6), wobei wenigstens die Auslassköpfe (6) mittels eines Antriebs gemeinsam als Gruppe in vertikaler Richtung bewegbar sind, wobei jeder Auslasskopf (6) eine vertikal orientierte Längsachse (LA) aufweist und eine sich entlang der Längsachse (LA) erstreckende und über die Zweigleitung (13a) mit der zentralen Zuleitung (13) verbundene Auslassleitung (7) zur Abgabe des Mediums in die Behälter (2) umfasst, wobei die Auslassleitung (7) zur Behandlung des Behälters (2) zumindest abschnittsweise in den in der Behälteraufnahme (4) hängend aufgenommenen Behälter (2) einführbar ist, **dadurch gekennzeichnet, dass**
jedem Auslasskopf (6) wenigstens ein steuerbarer Rückhaltemechanismus (10) zugeordnet ist, wobei die Vorrichtung (1) ferner wenigstens eine mit dem Rückhaltemechanismus (10) in kommunizierender Verbindung stehende Steuerung (11) aufweist und wobei der steuerbare Rückhaltemechanismus (10) zumindest ein Rückhalteelement (10a) aufweist und dazu ausgebildet ist, zumindest die Auslassleitung (7) eines jeweiligen Auslasskopfes (6) bedarfsgesteuert in einer Rückhalteposition (P1) zurückzuhalten, wobei
die Vorrichtung (1) ferner zumindest eine sensorische Erfassungseinheit (12) zur Erfassung der Anwesenheit von Behältern (2) in den Behälteraufnahmen (4) der Aufnahmevorrichtung (3) umfasst, wobei die sensorische Erfassungseinheit in kommunizierender Verbindung mit der Steuerung (11) steht.

2. Vorrichtung (1) nach Anspruch 1, **dadurch gekennzeichnet, dass** der Rückhaltemechanismus (10) jedes einzelnen Auslasskopfes (6) individuell steuerbar ist.

3. Vorrichtung (1) nach einem der Ansprüche 1 oder 2, **dadurch gekennzeichnet, dass** das Rückhalteelement (10a) dazu eingerichtet ist, mit einem an jedem Auslasskopf (6) vorgesehenen und mit der Auslassleitung (7) in Wirkverbindung stehenden Kopplungsmittel gesteuert in Eingriff zu gelangen, wobei durch Eingriff des Rückhalteelementes (10a) in das Kopplungsmittel die Rückhaltung der Auslassleitung (7) veranlasst wird.

4. Vorrichtung (1) nach einem der Ansprüche 1 bis 3, **dadurch gekennzeichnet, dass** die Auslassleitung (7) ein feststehendes Rohrstück (8) und ein bewegliches Auslassendstück (9) mit einer Auslassöffnung (9a) umfasst, wobei das Auslassendstück (9) in axialer Richtung entlang der Längsachse (LA) des jeweiligen Auslasskopfes (6) relativ zu dem Rohrstück (8) verschiebbar ist und wobei zur Rückhaltung der Auslassleitung (7) die axiale Verschiebung des Auslassendstücks (9) relativ zum Rohrstück (8) mittels des Rückhaltemechanismus (10) gesteuert erfolgt.

5. Vorrichtung (1) nach Anspruch 4, **dadurch gekennzeichnet, dass** die Auslassleitung (7) teleskopartig ausgebildet ist und das Auslassendstück (9) koaxial zu dem Rohrstück (8) angeordnet und axial verschiebbar zumindest teilweise in dem Rohrstück (8) aufgenommen ist.

6. Vorrichtung (1) nach Anspruch 5, **dadurch gekennzeichnet, dass** das Auslassendstück (9) über den Rückhaltemechanismus (10) durch axiale Verschiebung in vertikaler Richtung nach oben von wenigstens einer ausgefahrenen Position (P2) in das Rohrstück (8) einziehbar ist, und zwar in eine der Rückhalteposition (P1) entsprechende eingezogene Stellung, wobei eine Längserstreckung der Auslassleitung (7) entlang der Längsachse (LA) in der ausgefahrenen Position (P2) größer ist als in der eingezogenen Rückhalteposition (P1).

7. Vorrichtung (1) nach einem der vorhergehenden Ansprüche, **dadurch gekennzeichnet, dass** der Rückhaltemechanismus (10) über ein Stellglied rotativ oder linear betätigbar ist.

8. Vorrichtung (1) nach einem der vorhergehenden Ansprüche, **dadurch gekennzeichnet, dass** in jedem Auslasskopf (6) ein inneres Ventil zur geregelten Abgabe des Mediums über die Auslassöffnung (9a) vorgesehen ist, wobei das innere Ventil in einem geöffneten Zustand einen Abgabeströmungsweg für das Medium durch die Auslassleitung (7) freigibt und in einem geschlossenen Zustand den Abgabeströmungsweg verschließt.

9. Vorrichtung (1) nach Anspruch 8, **dadurch gekennzeichnet, dass** das innere Ventil derart eingerichtet und ausgebildet ist, dass der Abgabeströmungsweg in der Rückhalteposition (P1) der Auslassleitung (7) verschlossen ist.

10. Vorrichtung (1) nach einem der vorhergehenden Ansprüche 1 bis 7, **dadurch gekennzeichnet, dass** in jedem Auslasskopf (6) eine innere Drossel vorgesehen ist, wobei die innere Drossel derart eingerichtet und ausgebildet ist, dass die Abgabe des Mediums über die Auslassleitung (7) in der Rückhalteposition (P1) gedrosselt ist, derart, dass in der Rückhalteposition (P1) ein Fluss des Mediums durch die Auslassleitung (7) vermindert ist.

11. Vorrichtung (1) nach einem der vorhergehenden Ansprüche, **dadurch gekennzeichnet, dass** die sensorische Erfassungseinheit (12) durch wenigstens einen optischen Sensor oder durch einen Lagesensor gebildet ist.

12. Vorrichtung (1) nach einem der vorhergehenden Ansprüche, **dadurch gekennzeichnet, dass** zumindest die Auslassleitungen (7), insbesondere die Auslassleitungen (7) mit den verbundenen Zweigleitungen (13a) und der Zuleitung (13) derart höhenverstellbar ausgebildet sind, dass die Auslassöffnungen (9a) auf wenigstens eine vorgegebene Höhe und/oder stufenweise auf verschiedene Höhen oberhalb der Aufnahmevorrichtung (3) positionierbar sind.

13. Vorrichtung (1) nach einem der vorhergehenden Ansprüche, **dadurch gekennzeichnet, dass** die Vorrichtung als integrale Maschinenstation eines aseptischen Linearfüllers (20) ausgebildet ist und/oder dass die Vorrichtung (1) eine Vorrichtung zum Sterilisieren von Behältern ist.

14. Linearfüller (20) zum aseptischen Befüllen von Behältern (2) mit einem flüssigen Füllgut, aufweisend zumindest eine Transportvorrichtung (23) für den Transport der Behälter (2) in einer Transportrichtung (R) und zumindest eine Füllstation (21), wobei der Linearfüller (20) im Schrittbetrieb betrieben ist und wobei der Linearfüller (20) eine in Transportrichtung (R) vor der Füllstation (21) angeordnete Vorrichtung (1) zum Behandeln der Behälter (2) nach einem der Ansprüche 1 bis 13 aufweist, wobei die Vorrichtung (1) eine Behältersterilisierung des Linearfüllers (20) bildet.

15. Verfahren zum Behandeln von gruppenweise entlang eines Förderwegs im Taktbetrieb transportierten Behältern (2) mit einem Medium, bei dem die Behälter (2) mittels einer linearen Aufnahmevorrichtung (3) mit mehreren Behälteraufnahmen (4) in Form einer geordneten Behältergruppe (BG) mit vorgegebener Anzahl an Behältern (2) hängend gehalten und in einer Behandlungsstation einer Vorrichtung (1) unterhalb einer Behandlungseinrichtung (5) positioniert werden, wobei die Behandlungseinrichtung (5) eine Zuleitung (13) für das Medium und mehrere über Zweigleitungen (13a) an die Zuleitung angeschlossene Auslassköpfe (6) umfasst und wobei jedem Behälter (2) der Behältergruppe (BG) ein Auslasskopf (6) zugeordnet ist, wobei bei dem Verfahren die Auslassköpfe (6) zur Behandlung als Gruppe gemeinsam mittels eines entsprechenden Antriebs durch eine Hub-Senkbewegung in vertikaler Richtung nach unten bewegt und dadurch den zugeordneten Behältern (2) derart angenähert werden, dass eine bei jedem Auslasskopf (6) vorgesehene und über die Zweigleitung (13a) mit der Zuleitung (13) verbundene Auslassleitung (7) für die Abgabe des Mediums (7) über eine jeweilige Behältermündung wenigstens teilweise in den zugeordneten Behälter (2) eingeführt wird, **dadurch gekennzeichnet, dass** die Auslassleitung (7) eines jeweiligen Auslasskopfes (6) mittels eines durch eine Steuerung (11) gesteuerten Rückhaltemechanismus (10) bedarfsgesteuert in einer Rückhalteposition (P1) zurückgehalten wird,
wobei besagte Vorrichtung (1) zumindest eine sensorische Erfassungseinheit (12) zur Erfassung der Anwesenheit von Behältern (2) in den Behälteraufnahmen (4) der Aufnahmevorrichtung (3) umfasst, wobei die sensorische Erfassungseinheit in kommunizierender Verbindung mit der Steuerung (11) steht.

16. Verfahren nach Anspruch 15, **dadurch gekennzeichnet, dass** jede Auslassleitung (7) individuell und bedarfsgesteuert in der Rückhalteposition (P1) zurückgehalten wird.

17. Verfahren nach Anspruch 15 oder 16, **dadurch gekennzeichnet, dass** die Auslassleitung (7) in der Rückhalteposition (P1) dort zurückgehalten wird, wo ein Behälter (2) in der Behältergruppe (BG) fehlt, nämlich wenn aufgrund eines fehlenden Behälters (2) die dem jeweiligen Auslasskopf (6) zugeordnete Behälteraufnahme (4) der linearen Aufnahmevorrichtung (3) unbelegt ist.

18. Verfahren nach einem der Ansprüche 15 bis 17, **dadurch gekennzeichnet, dass** es sich um ein Verfahren zum Sterilisieren von Behältern (2) handelt, wobei zumindest in der Behandlungsstation der Vorrichtung (1) wenigstens in einem Bereich oberhalb der Aufnahmevorrichtung (3) eine Aseptikzone (S) ausgebildet und aufrechterhalten wird, und zwar zumindest in dem Bereich, in dem die Behältermündungen der hängend gehaltenen Behälter (2) angeordnet sind, wobei die Auslassleitung (7) bedarfsgesteuert derart zurückgehalten wird, dass die zurückgehaltene Auslassleitung (7) in der Rückhalteposition (P1) vollständig in der Aseptikzone (S) angeordnet ist.

19. Verfahren nach einem der Ansprüche 15 bis 18, **dadurch gekennzeichnet, dass** die Auslassleitung (7) zumindest ein feststehendes Rohrstück (8) und ein bewegliches Endstück (9) umfasst, wobei zum Einnehmen der Rückhalteposition (P1) der Auslassleitung (7) das Endstück (9) in axialer Richtung entlang der Längsachse (LA) des jeweiligen Auslasskopfes (6) relativ zu dem Rohrstück (8) verschoben wird.

## Claims

1. Apparatus (1) for treating containers (2) being transported in groups along a conveying path in cyclic operation with a medium, in particular a gaseous, vaporous, or liquid medium, comprising at least one linear holding apparatus (3) with a plurality of container holders (4) for the suspended holding of the containers (2), at least one treatment station, comprising at least one treatment device (5) with a supply line (13) for the medium and a plurality of outlet heads (6), connected by respective branch lines (13a) to the supply line (13), wherein at least the outlet heads (6) can be moved by means of a drive together as a group in a vertical direction, wherein each outlet head (6) has a vertically oriented longitudinal axis (LA) and an outlet line (7), extending along the longitudinal axis (LA) and connected via the branch line (13a) to the central supply line (13), for discharging the medium into the containers (2), wherein the outlet line (7) for treating the container (2) can be introduced at least in sections into the container (2) held suspended in the container holder (4), **characterised in that**
at least one controllable restraining mechanism (10) is assigned to each outlet head (6), wherein the apparatus (1) further comprises at least one control means (11) which is in communicative connection with the restraining mechanism (10), and wherein the controllable restraining mechanism (10) comprises at least one restraining element (10a) and is configured such as to restrain at least the outlet line (7) of a respective outlet head (6), in a controlled manner as required, in a restraining position (P1), wherein
the apparatus (1) further comprises at least one sensory detection unit (12) for detecting the presence of containers (2) in the container holders (4) of the holding device (3), wherein the sensory detection unit is in communicative connection with the control means (11).

2. Apparatus (1) according to claim 1, **characterised in that** the restraining mechanism (10) of each individual outlet head (6) can be controlled individually.

3. Apparatus (1) according to any one of claims 1 or 2, **characterised in that** the restraining element (10a) is configured such as to move into engagement in a controlled manner with a coupling means, provided at each outlet head (6) and in operational connection with the outlet line (7), wherein, by the engagement of the restraining element (10a) into the coupling means, the restraining of the outlet line (7) is arranged.

4. Apparatus (1) according to any one of claims 1 to 3, **characterised in that** the outlet line (7) comprises a fixed tube piece (8) and a movable outlet end piece (9) with an outlet opening (9a), wherein the outlet end piece (9) can be displaced along the longitudinal axis (LA) of the respective outlet head (6) relative to the tube piece (8), and wherein, for the restraint of the outlet line (7), the axial displacement of the outlet end piece (9) relative to the tube piece (8) takes place in a controlled manner by means of the restraining mechanism (10).

5. Apparatus (1) according to claim 4, **characterised in that** the outlet line (7) is configured as telescopic, and the outlet end piece (9) is arranged coaxially to the tube piece (8), and is held at least partially in the tube piece (8) in an axially displaceable manner.

6. Apparatus (1) according to claim 5, **characterised in that** the outlet end piece (9) can be drawn in by means of the restraining mechanism (10), by axial displacement in a vertical direction upwards, from at least one extended position (P2) into the tube piece (8), and specifically into a retracted position corresponding to the restraining position (P1), wherein a longitudinal extension of the outlet line (7) along the longitudinal axis (LA) in the extended position (P2) is greater than in the retracted restraint position (P1).

7. Apparatus (1) according to any one of the preceding claims, **characterised in that** the restraining mechanism (10) can be actuated by means of an actuator in a rotational or linear manner.

8. Apparatus (1) according to any one of the preceding claims, **characterised in that** in each outlet head (6) an inner valve is provided for the regulated discharge of the medium via the outlet opening (9a), wherein the inner valve, in an opened state, clears a discharge flow path for the medium through the outlet line (7) and in a closed state closes the discharge flow path.

9. Apparatus (1) according to claim 8, **characterised in that** the inner valve is configured and formed in such a way that the discharge flow path is closed in the restraining position (P1) of the outlet line (7).

10. Apparatus (1) according to any one of the preceding claims 1 to 7, **characterised in that** in each outlet head (6) an inner choke is provided, wherein the inner choke is configured and formed in such a way that the discharge of the medium via the outlet line (7) in the restrained position (P1) is choked in such a way that, in the restrained position (P1) a flow of the medium through the outlet line (7) is reduced.

11. Apparatus (1) according to any one of the preceding claims, **characterised in that** the sensory detection unit (12) is formed by at least one optical sensor or by a location sensor.

12. Apparatus (1) according to any one of the preceding claims, **characterised in that** at least the outlet lines (7), in particular the outlet lines (7), with the connected branch lines (13a) and the supply line (13) are configured to be height adjustable in such a way that the outlet openings (9a) can be positioned at least at one predetermined height and/or in steps at different heights above the holding apparatus (3).

13. Apparatus (1) according to any one of the preceding claims, **characterised in that** the apparatus is configured as an integral machine station of an aseptic linear filler (20), and/or that the apparatus (1) is an apparatus for the sterilising of containers.

14. Linear filler (20) for the aseptic filling of containers (2) with a liquid filling material, comprising at least one transport apparatus (23) for the transport of the containers (2) in a transport direction (R) and at least one filling station (21), wherein the linear filler (20) is operated in step operation, and wherein the linear filler (20) comprises an apparatus (1) for treating containers (2), arranged in the transport direction (R) upstream of the filling station (21), according to any one of claims 1 to 13, wherein the apparatus (1) forms a container sterilisation element of the linear filler (20).

15. Method for treating containers (2), being transported in groups along a conveying path in cyclic operation, with a medium, wherein the containers (2) are held suspended by means of a linear holding apparatus (3) with a plurality of container holders (4) in the form of an ordered container group (BG) with a predetermined number of containers (2), and are positioned in a treatment station of an apparatus (1) beneath a treatment device (5), wherein the treatment device (5) comprises a supply line (13) for the medium and a plurality of outlet heads (6) connected by means of branch lines (13a) to the supply line, and wherein an outlet head (6) is assigned to each container (2) of the container group (BG), wherein, with the method, the outlet heads (6) for the treatment are moved as a group by means of a corresponding drive, by a stroke lowering movement in a vertical direction downwards, and, as a result, are brought close to the assigned containers (2) in such a way that an outlet line (7), provided at each outlet head (6) and connected via the branch line (13a) to the supply line (13), is introduced at least partially into the assigned container (2) for the discharge of the medium (7) via a respective container mouth, **characterised in that** the outlet line (7) of a respective outlet head (6) is restrained in a restraining position (P1) in a controlled manner as required, by means of a restraining mechanism (10) which is controlled by a control means (11),
wherein the said apparatus (1) comprises at least one sensory detection unit (12) for detecting the presence of containers (2) in the container holders (4) of the holding apparatus (3), wherein the sensory detection unit is in communicative communication with the control means (11).

16. Method according to claim 15, **characterised in that** each outlet line (7) is restrained in the restraint position (P1) individually and controlled as required.

17. Method according to claim 15 or 16, **characterised in that** the outlet line (7) in the restraining position (P1) is restrained at the place at which a container (2) is missing in the container group (BG), namely, if due to a missing container (2), the container holder (4) of the linear holding apparatus (3) assigned to the respective outlet head (6) is unoccupied.

18. Method according to any one of claims 15 to 17, **characterised in that** this relates to a method for the sterilising of containers (2), wherein at least in the treatment station of the apparatus (1), at least in a region above the holding apparatus (3), an aseptic zone (S) is formed and maintained, and specifically at least in the region in which are arranged the container mouths of the containers (2) being held suspended, wherein the outlet line (7) is restrained in a controlled manner as required, in such a way that the restrained outlet line (7) in the restrained position (P1) is arranged entirely in the aseptic zone (S).

19. Method according to any one of claims 15 to 18, **characterised in that** the outlet line (7) comprises at least one fixed tube piece (8) and a movable end piece (9), wherein, in order to adopt the restrained or retracted position (P1) of the outlet line (7), the end piece (9) is moved in the axial direction along the longitudinal axis (LA) of the respective outlet head (6), relative to the tube piece (8).

## Revendications

1. Dispositif (1) pour le traitement de contenants (2) transportés par groupes le long d'une voie de transport en cadence avec un milieu, en particulier un milieu sous forme de gaz, de vapeur ou de liquide, présentant au moins un dispositif de réception (3) linéaire avec plusieurs logements de contenant (4) pour la réception en suspension des contenants (2), au moins une station de traitement comprenant au moins un dispositif de traitement (5) avec une conduite d'amenée (13) pour le milieu ainsi qu'avec plusieurs têtes de sortie (6) raccordées par le biais de conduites dérivées (13a) respectives à la conduite d'amenée (13), dans lequel au moins les têtes de sortie (6) sont mobiles au moyen d'un entraînement de manière commune en tant que groupe dans le sens vertical, dans lequel chaque tête de sortie (6) présente un axe longitudinal (LA) orienté verticalement et comprend une conduite de sortie (7) s'étendant le long de l'axe longitudinal (LA) et reliée par le biais de la conduite dérivée (13a) à la conduite d'amenée (13) centrale pour la distribution du milieu dans les contenants (2), dans lequel la conduite de sortie (7) peut être introduite pour le traitement du contenant (2) au moins par sections dans le contenant (2) reçu en suspension dans le logement de contenant (4), **caractérisé en ce que**
au moins un mécanisme de retenue (10) commandable est associé à chaque tête de sortie (6), dans lequel le dispositif (1) présente de plus au moins une commande (11) se trouvant en liaison de communication avec le mécanisme de retenue (10) et dans lequel le mécanisme de retenue (10) commandable présente au moins un élément de retenue (10a) et est réalisé afin de retenir au moins la conduite de sortie (7) d'une tête de sortie (6) respective si besoin dans une position de retenue (P1), dans lequel
le dispositif (1) comprend de plus au moins une unité de détection (12) par capteur pour la détection de la présence de contenants (2) dans les logements de contenant (4) du dispositif de réception (3), dans lequel l'unité de détection par capteur est en liaison de communication avec la commande (11).

2. Dispositif (1) selon la revendication 1, **caractérisé en ce que** le mécanisme de retenue (10) de chaque tête de sortie (6) individuelle est commandable individuellement.

3. Dispositif (1) selon l'une quelconque des revendications 1 ou 2, **caractérisé en ce que** l'élément de retenue (10a) est conçu afin de parvenir en prise de manière commandée avec un moyen de couplage prévu au niveau de chaque tête de sortie (6) et se trouvant en liaison active avec la conduite de sortie (7), dans lequel la retenue de la conduite de sortie (7) est provoquée par prise de l'élément de retenue (10a) dans le moyen de couplage.

4. Dispositif (1) selon l'une quelconque des revendications 1 à 3, **caractérisé en ce que** la conduite de sortie (7) comprend une pièce tubulaire (8) fixe et une pièce d'extrémité de sortie (9) mobile avec une ouverture de sortie (9a), dans lequel la pièce d'extrémité de sortie (9) est coulissante dans le sens axial le long de l'axe longitudinal (LA) de la tête de sortie (6) respective par rapport à la pièce tubulaire (8) et dans lequel le coulissement axial de la pièce d'extrémité de sortie (9) par rapport à la pièce tubulaire (8) est effectué de manière commandée au moyen du mécanisme de retenue (10) pour la retenue de la conduite de sortie (7).

5. Dispositif (1) selon la revendication 4, **caractérisé en ce que** la conduite de sortie (7) est réalisée de manière télescopique et la pièce d'extrémité de sortie (9) est agencée coaxialement à la pièce tubulaire (8) et est reçue de manière axialement coulissante au moins partiellement dans la pièce tubulaire (8).

6. Dispositif (1) selon la revendication 5, **caractérisé en ce que** la pièce d'extrémité de sortie (9) est rétractable par le biais du mécanisme de retenue (10) par coulissement axial dans le sens vertical vers le haut d'au moins une position sortie (P2) dans la pièce tubulaire (8), et ce dans une position rétractée correspondante à la position de retenue (P1), dans lequel une étendue longitudinale de la conduite de sortie (7) le long de l'axe longitudinal (LA) dans la position sortie (P2) est plus grande que dans la position de retenue (P1) rétractée.

7. Dispositif (1) selon l'une quelconque des revendications précédentes, **caractérisé en ce que** le mécanisme de retenue (10) peut être actionné par le biais d'un organe de réglage de manière rotative ou linéaire.

8. Dispositif (1) selon l'une quelconque des revendications précédentes, **caractérisé en ce qu'**une soupape intérieure est prévue dans chaque tête de sortie (6) pour la distribution régulée du milieu par le biais de l'ouverture de sortie (9a), dans lequel la soupape intérieure libère dans un état ouvert une voie d'écoulement de distribution pour le milieu par la conduite de sortie (7) et ferme dans un état fermé la voie d'écoulement de distribution.

9. Dispositif (1) selon la revendication 8, **caractérisé en ce que** la soupape intérieure est conçue et réalisée de telle manière que la voie d'écoulement de distribution soit fermée dans la position de retenue (P1) de la conduite de sortie (7).

10. Dispositif (1) selon l'une quelconque des revendications précédentes 1 à 7, **caractérisé en ce qu'**un étranglement intérieur est prévu dans chaque tête de sortie (6), dans lequel l'étranglement intérieur est conçu et réalisé de telle manière que la distribution du milieu soit étranglée par le biais de la conduite de sortie (7) dans la position de retenue (P1) de telle manière qu'un flux du milieu par la conduite de sortie (7) soit réduit dans la position de retenue (P1).

11. Dispositif (1) selon l'une quelconque des revendications précédentes, **caractérisé en ce que** l'unité de détection (12) par capteur est formée par au moins un capteur optique ou par un capteur de position.

12. Dispositif (1) selon l'une quelconque des revendications précédentes, **caractérisé en ce qu'**au moins les conduites de sortie (7), en particulier les conduites de sortie (7) sont réalisées avec les conduites dérivées (13a) reliées et la conduite d'amenée (13) de manière réglable en hauteur de telle manière que les ouvertures de sortie (9a) puissent être positionnées à au moins une hauteur prédéfinie et/ou progressivement à différentes hauteurs au-dessus du dispositif de réception (3).

13. Dispositif (1) selon l'une quelconque des revendications précédentes, **caractérisé en ce que** le dispositif est réalisé en tant que station de machine intégrale d'une remplisseuse linéaire (20) aseptisée et/ou **en ce que** le dispositif (1) est un dispositif de stérilisation de contenants.

14. Remplisseuse linéaire (20) pour le remplissage aseptisé de contenants (2) avec un produit de remplissage liquide, présentant au moins un dispositif de transport (23) pour le transport des contenants (2) dans un sens de transport (R) et au moins une station de remplissage (21), dans lequel la remplisseuse linéaire (20) fonctionne par à-coups et dans lequel la remplisseuse linéaire (20) présente un dispositif (1) de traitement des contenants (2) selon l'une quelconque des revendications 1 à 13 agencé dans le sens de transport (R) avant la station de remplissage (21), dans lequel le dispositif (1) forme une stérilisation de contenant de la remplisseuse linéaire (20).

15. Procédé de traitement de contenants (2) transportés par groupes le long d'une voie de transport en cadence avec un milieu, pour lequel les contenants (2) sont maintenus en suspension au moyen d'un dispositif de réception (3) linéaire avec plusieurs logements de contenant (4) sous la forme d'un groupe de contenant (BG) ordonné avec un nombre prédéfini de contenants (2) et positionnés dans une station de traitement d'un dispositif (1) en dessous d'un dispositif de traitement (5), dans lequel le dispositif de traitement (5) comprend une conduite d'amenée (13) pour le milieu et plusieurs têtes de sortie (6) raccordées par le biais de conduites dérivées (13a) à la conduite d'amenée et dans lequel une tête de sortie (6) est associée à chaque contenant (2) du groupe de contenant (BG), dans lequel pour le procédé les têtes de sortie (6) sont déplacées vers le bas pour le traitement en tant que groupe de manière commune au moyen d'un entraînement correspondant par un mouvement de levage et d'abaissement dans le sens vertical et ainsi sont approchées des contenants (2) associés de telle manière qu'une conduite de sortie (7) reliée par le biais de la conduite dérivée (13a) à la conduite d'amenée (13) et prévue pour chaque tête de sortie (6) pour la distribution du milieu (7) par le biais d'une embouchure de contenant respective soit introduite au moins partiellement dans le contenant (2) associé, **caractérisé en ce que** la conduite de sortie (7) d'une tête de sortie (6) respective est retenue au moyen d'un mécanisme de retenue (10) commandé par une commande (11) si besoin dans une position de retenue (P1),
dans lequel ledit dispositif (1) comprend au moins une unité de détection (12) par capteur pour la détection de la présence de contenants (2) dans les logements de contenant (4) du dispositif de réception (3), dans lequel l'unité de détection par capteur est en liaison de communication avec la commande (11).

16. Procédé selon la revendication 15, **caractérisé en ce que** chaque conduite de sortie (7) est retenue individuellement et si besoin dans la position de retenue (P1).

17. Procédé selon la revendication 15 ou 16, **caractérisé en ce que** la conduite de sortie (7) est retenue dans la position de retenue (P1) là où un contenant (2) fait défaut dans le groupe de contenant (BG), à savoir lorsqu'en raison d'un contenant (2) manquant, le logement de contenant (4) associé à la tête de sortie (6) respective du dispositif de réception (3) linéaire est inoccupé.

18. Procédé selon l'une quelconque des revendications 15 à 17, **caractérisé en ce qu'**il s'agit d'un procédé de stérilisation de contenants (2), dans lequel une zone aseptisée (S) est réalisée et maintenue au moins dans la station de traitement du dispositif (1), au moins dans une zone au-dessus du dispositif de réception (3), et ce au moins dans la zone, dans laquelle les embouchures de contenant des contenants (2) maintenus en suspension sont agencées, dans lequel la conduite de sortie (7) est retenue si besoin de telle manière que la conduite de sortie (7) retenue soit agencée dans la position de retenue (P1) complètement dans la zone aseptisée (S).

19. Procédé selon l'une quelconque des revendications 15 à 18, **caractérisé en ce que** la conduite de sortie (7) comprend au moins une pièce tubulaire (8) fixe et une pièce d'extrémité (9) mobile, dans lequel la pièce d'extrémité (9) est coulissée dans le sens axial le long de l'axe longitudinal (LA) de la tête de sortie (6) respective par rapport à la pièce tubulaire (8) pour occuper la position de retenue (P1) de la conduite de sortie (7).
